# EUROPEAN PATENT SPECIFICATION

(11) **EP 0 523 013 B1**
(45) Date of publication and mention of the grant of the patent: **21.12.1994**
(21) Application number: 92830346.0
(22) Date of filing: 02.07.1992
(51) Int. Cl.: A61K 31/435

(54) **Use of benzimidazoline-2-oxo-1-carboxylic acid derivatives in the treatment of organic mental diseases**
Verwendung von Benzimidazolin-2-oxo-1-Carbonsäurederivaten bei der Behandlung organischer Gehirnerkrankungen
Utilisation des dérivés de l'acide benzimidazoline-2-oxo-1-carboxylique dans le traitement des maladies mentales organiques

(30) Priority: 04.07.1991 IT MI911845
(43) Date of publication of application: 13.01.1993
(73) Proprietor: BOEHRINGER INGELHEIM ITALIA S.p.A., 50123 Firenze (IT)
(72) Inventor: Brambilla, Alessandro, Milano (IT); Turconi, Marco, Voghera (Pavia) (IT); Schiantarelli, Pierino, Piacenza (IT); Borsini, Franco, Prato (Firenze) (IT); Ladinsky, Herbert, Milano (IT)
(74) Representative: Aimi, Luciano

(56) References cited:
- EP-A- 0 190 920
- EP-A- 0 279 990
- EP-A- 0 309 423
- JOURNAL OF MEDICINAL CHEMISTRY, vol. 33, no. 8, 1990, pages 2101-2108, American Chemical Society; M. TURCONI et al.: "Synthesis of a new class of 2,3-dihydro-2-oxo-1H-benzimidazole-1-carboxylic acid derivatives as highly potent 5-HT3 receptor antagonists"
- NAUNYN-SCHMIEDEBERG'S ARCHIVES OF PHARMACOLOLOGY, vol. 343, no. 3, March 1991, pages 245-251, Springer-Verlag; A. DUMUIS et al.: "Azabicycloalkyl benzimidazolone derivatives as a novel class of potent agonists at the 5-HT4 receptor positively coupled to adenylate cyclase in brain"
- MEDICINAL RESEARCH REVIEWS, vol. 10, no. 4, October-December 1990, pages 441-475, John Wiley & Sons, Inc.; G.J. KILPATRICK et al.: "5-HT3 receptors"

## Description

The present invention relates to a new medical use of benzimidazoline-2-oxo-1-carboxylic acid derivatives and physiologically acceptable acid addition salts and solvates thereof in the treatment of organic mental disorders and to pharmaceutical compositions containing them.
Among the untreatable forms of cognitive disorders, senile dementia of Alzheimer type, age-associated memory impairment and multi-infarct dementia are the clinical syndromes which present the greatest challenge. Various pharmacological strategies are currently used to treat these cognitive disorders in human. They foresee the use of nootropics, vasodilators, metabolic enhancers and psychostimulants. Not one of these agents improves cognition unequivocally in demented patients. (Medicinal Research Reviews, Vol. 8, No. 3, 353-391, 1988).
Compounds known to possess cognitive enhancing properties in animals may be used in the treatment of dementia conditions. Cholinergic agents, calcium antagonists, neuropeptides and phospholipids have been reported to facilitate memory in animals.

European Patent Application no. 309423 described a new class of benzimidazoline-2-oxo-1-carboxylic acid derivatives useful as serotonin (5-HT) receptor antagonists. According to the major role played by 5-HT in both the peripheral nervous system (PNS) and in the central nervous system (CNS), the above compounds were claimed for the treatment of nausea and vomiting, induced by the administration of anticancer cytotoxic drugs and radiations, e.g. X-ray radiations. Non limitative examples of cytotoxic drugs include cisplatin, doxorubicin, cyclophosphamide. The above benzimidazoline derivatives were also claimed for the treatment of gastrointestinal motor disturbances, such as delayed gastric emptying, dyspepsia, flatulence, oesophageal reflux, peptic ulcer, constipation and irritable bowel syndrome. European Patent Application no. 309423 also described the usefulness of benzimidazoline-2-oxo-1-carboxylic acid derivatives in the treatment of anxiety, psychoses, migraine, cluster headaches and trigeminal neuralgia.

EP-A-0279990 relates to the use, inter alia, of compounds containing a substituted endo-azabicycloalkyl moiety in their molecules for the treatment of cognitive disorders.
EP-A-0190920 discloses the use of bridged arylamidoazabicycloalkanes for the treatment of memory deficiencies.
Compounds similar to those of the above cited EP-A-s and others are also named as 5-HT₃ receptor antagonists in Med. Res. Rev., 10(4), 1990, pages 441-475.

It has now been surprisingly discovered, and this is the object of the present invention, that a class of compounds described in the above mentioned EP-A-0309423, i.e. the compounds of the general formula (I) 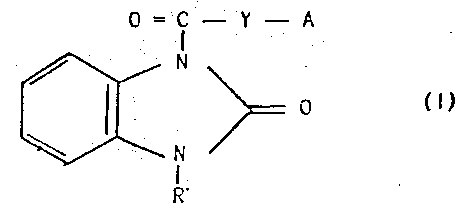
wherein R represents a hydrogen atom, C₁₋₆ alkyl, C₂₋₆ alkenyl or C₂₋₆ alkynyl, Y is NH, A is 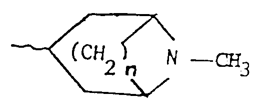
wherein n is 2; and physiologically acceptable acid addition salts and solvates thereof, are advantageous for the treatment of organic mental disorders such as amnestic syndromes, primary degeneration dementia (AD), benign senescent forgetfulness, multi infarct dementia, Korsakoff's syndrome, geriatric depression and attentional deficit disorders.

The effectiveness of the compounds of general formula I in the treatment of organic mental diseases may be demonstrated in the ability to antagonize the scopolamine induced impairment in the passive avoidance test in rat.

For the pharmaceutical use the compounds of general formula (I) are used as such or under the form of physiologically acceptable acid addition salts. The term "acid addition salt" includes salts with inorganic or organic acids. Physiologically acceptable acids used for the salification include, for example, maleic, citric, tartaric, fumaric, methansulphonic, hydrochloric, hydrobromic, idroiodic, nitric, sulphuric, phosphoric, acetic, benzoic, ascorbic acid, preferably hydrochloric acid.

The compounds of general formula (I) and their physiologically acceptable acid addition salts may be also employed as physiologically acceptable solvates, such as for example hydrates.

Preferred compounds according to the present invention are the following:
N-(endo-8-methyl-8-azabicyclo[3.2.1]oct-3-yl)-2,3-dihydro-2-oxo-!H-benzimidazole-1-carboxamide (compound 1)
N-(endo-8-methyl-8-azabicyclo[3.2.1]oct-3-yl)2,3-dihydro-3-ethyl-2-oxo-1H-benzimidazole-1-carboxamide (compound 5)
N-(endo-8-methyl-8-azabicyclo[3.2.1]oct-3-yl)-2,3-dihydro-3-(1-methyl)ethyl-2-oxo-1H-benzimidazole-1-carboxamide (compound 6)

### PHARMACOLOGY

Male Sprague Dawley rats weighing 175-200 g were used. The animals were kept in a regulated environment (20 ± 1°C, 50-55% humidity and 12 hours light-dark cycle, light on at 7.00 a.m.) and maintained with standard pellet diet.
The apparatus used for the passive avoidance test was composed of a dark compartment (30x30x30 cm) with a grid floor and a compartment (30x30x30 cm) illuminated from above by a 20 W daylight lamp, the 2 compartments being separated by a guillotine-type door (10x8 cm). Scrambled electric shocks were delivered to the grid floor by an isolated stimulator.

Rats were divided in groups of 10 animals.
a) Training: each rat was placed gently in the lighted compartment and 10 sec later the door was opened. As soon as the rat had moved into the dark compartment and the door had been shut, a 1.6 mA foot-shock was applied for 1 sec. The rat was immediately removed from the apparatus and returned to the home cage.
b) Retention: the retention test was carried out 24 hours later by an observer unaware of the given treatment. Each rat was placed into the lighted compartment again and the step-through latency was recorded. The test is stopped when the rat enters the dark compartment or when it fails to do so in less than 180 sec.
   The impairment induced by 0.75 mg/kg of scopolamine was used to examine the antiamnestic effects of test compounds. For this purpose the compounds were administered i.p. min before scopolamine, i.e. 45 min before training.
   The experimental data were evaluated statistically by means of Mann Whitney-U test, comparing all treatments, including controls, with scopolamine group.

### RESULTS

Nonshocked rats usually found the entrance into the dark compartment within few sec and entered it without hesitation, either during training (19 ± 4.3 sec) and the retention test (12 ± 3.3 sec). On the contrary, rats of the control groups, receiving electric shocks, showed prolonged latency to re-enter the dark compartment and most of them (about 80%) remained in the lighted compartment.
Scopolamine given 30 min before training, in a dose-dependent fashion, significantly decreased the step-through latency in the retention test. The amnestic effect caused by scopolamine was inhibited by the test compounds. None of the tested drugs modified latencies during the training session. The results are reported in the table 1.

**TABLE 1**

| Effect of the test compounds on scopolamine-induced impairment of passive avoidance response in rats. | |
|---|---|
| TREATMENT | LATENCY (SEC) |
| Control | 167 ± 8.8 ** |
| Scopolamine | 66 ± 18.7 |
| Compound 1 0.01 mg/kg | 136 ± 16.0 ** |
| Compound 1 0.03 mg/kg | 109 ± 17.0 * |
| Compound 5 0.03 mg/kg | 113 ± 17.0 ** |
| Compound 6 0.03 mg/kg | 140 + 16 ** |
| Values represent mean ± S.E.M from 10 animals. Test compounds were given i.p. 15 min before scopolamine, which was administered at the dose of 0.75 mg/kg i.p. 30 min before the training session. | |

| | |
|---|---|
| * p<0.05 and | |
| ** p<0.01 as compared with scopolamine group (Mann Whitney-U test). | |

Pharmaceutical compositions for use according to the present invention comprise as active ingredient, an effective amount of a compound of general formula (I) or physiologically acceptable acid addition salts or solvates thereof in association with one or more pharmaceutical carriers, diluents or excipients.

The compositions may be formulated in conventional manner, for oral, parenteral, rectal or transdermal administration or in a form suitable for administration by inhalation or insufflation (either through the mouth or the nose).

For oral administration, the pharmaceutical compositions may be, for example, in the form of, tablets, (including sustained release tablets) or capsules prepared in conventional manner with pharmaceutically. acceptable excipients such as corn starch, polyvinylpyrrolidone, lactose, microcrystalline cellulose, magnesium stearate, talc, potato starch, sodium lauryl sulphate. Liquid preparations for oral administration may be, for example, in the form of solutions, syrups or suspensions which may be prepared in conventional manner with pharmaceutically acceptable additives such as sorbitol syrup, cellulose derivatives, lecithin, almond oil, methyl p-hydroxybenzoate, and if desired, buffer salts, flavouring, colouring and sweetening agents.

Compounds of formula (I) may be formulated for parenteral administration by injection, e.g. by bolus injection or continuous infusion. Compositions for injection may be presented in unit dosage form, e.g. in ampoules or in multi-dose containers. The compositions may be in the form of suspensions, solutions or emulsions in oily or aqueous vehicles. Alternatively, the active ingredient may be in powder form for constitution with a suitable vehicle, e.g. sterile pyrogen water, before use.

For rectal administration the pharmaceutical compositions may be, for example, in the form of suppositories containing conventional suppository bases, such as cocoa butter or other glycerides.

Besides the above described compositions, the compounds of formula (I) may also be formulated as a depot composition. Such long acting compositions may be administered by implantation (for example subcutaneously, transcutaneously or intramuscularly) or by intramusclular injection. Thus, these preparations may be, for example, formulated with suitable polymeric or hydrophobic materials or ion exchange resins.

The compositions are advantageously formulated in dosage unit; each dosage unit being adapted to supply a single dose of the active ingredient. Each dosage unit may conveniently contain from 0.005 mg to 30 mg, preferably from 0.05 mg to 10 mg of the active ingredient.

The compounds of general formula I may be prepared according to the processes described in the European patent application no. 309423 and the following examples illustrate their preparation:

### EXAMPLE 1

### N-(endo-8-methyl-8-azabicyclo[3.2.1]oct-3-yl)-2,3-dihydro-2-oxo-1H-benzimidazole-1-carboxamide

### (Compound 1)

2,3-Dihydro-2-oxo-1H-benzimidazole-1-carbonyl chloride (1.5 g) was dissolved in tetrahydrofurane (40 ml) and to that solution a solution of endo-8-methyl-8-azabicyclo[3.2.1]octan-3-amine, dissolved in tetrahydrofurane (5 ml), was added dropwise at room temperature. When the addition was over a solid separated and the reaction mixture was stirred for 30 minutes, concentrated to dryness and taken up in diluted HCl. The aqueous phase was washed with ethyl acetate, made basic with a saturated sodium carbonate and again extracted. The latter organic layers were concentrated to dryness giving 0.7 g of the crude product.
By crystallization form acetonitrile 0.17 g of the pure product were obtained. M.p. 205-207° C.

| | | | | |
|---|---|---|---|---|
| Analysis | Found % | C 62.83 | H 6.75 | N 18.01 |
| C₁₆H₂₀N₄O₂ | Calc. % | C 63.98 | H 6.71 | N 18.65. |

The hydrochloride acid salt was obtained by dissolving the base in absolute ethanol and by adding gaseous hydrogen chloride. M.p. 292° C.

| | | | | |
|---|---|---|---|---|
| Analysis | Found % | C 56.94 | H 6.02 | N 16.54 |
| C₁₆H₂₀N₄O₂.HCl | Calc. % | C 57.05 | H 5.98 | N 16.63. |

### EXAMPLE 2

### N-(endo-8-methyl-8-azabicyclo[3.2.1]oct.-3-yl)-2,3-dihydro-3-ethyl-2-oxo-1H-benzimidazole-1-carboxamide

### (Compound 5)

N-(endo-8-methyl-8-azabicyclo[3.2.1]oct.-3-yl)-2,3-dihydro-2-oxo-1H-benzimidazole-1-carboxamide hydrochloride (8.0 g) was suspended in dry dimethylformamide (120 ml) and 1.42 g of sodium hydride, as 80% dispersion in mineral oil, was added portionwise. The resulting reaction mixture was stirred at room temperature for 3 hours, then ethyl iodide (3.7 g) was added in 15 minutes. The suspension was stirred for further three hours at room temperature. Diluted hydrochloric acid was cautiously added, the final mixture was poured onto crushed ice. The aqueous phase was washed with ethyl acetate and then treated with sodium carbonate until pH 10. The raw compound was extracted into ethylacetate, washed with water and concentrated to dryness. Pure N-endo-8-methyl-8-azabicyclo[3.2.1]oct-3-yl)-2,3-dihydro-3-ethyl-2-oxo-1H-benzimidazole-1-carboxamide as afforded by crystallizing the raw compound as its hydrochloric acid salt from hot acetone. Yield 4.5 g (52%) M.p. 242-244° C.

| | | | | |
|---|---|---|---|---|
| Analysis | Found % | C 58.35 | H 7.06 | N 15.01 |
| C₁₆H₂₀N₄O₂.HCl | Calc. % | C 59.25 | H 6.91 | N 15.36 |

### EXAMPLE 3

The procedure described in Example 2 was repeated using N-endo-8-methyl-8-azabicyclo[3.2.1]oct.-3-yl)-2,3-dihydro-2-oxo-1H-benximidazole-1-carboxamide (compound 1) as the starting compound and selected alkyl halides as electrophylic reagents to afford the following compounds:

### N-(endo-8-methyl-8-azabicyclo[3.2.1]oct.-3-yl)-2,3-dihydro-3-(1-methyl)ethyl-2-oxo-1H-benzimidazole-1-carboxamide, hydrochloric acid salt

### (Compound 6)

M.p. 117-120° C.

| | | | | |
|---|---|---|---|---|
| Analysis | Found % | C 58.97 | H 7.34 | N 14.23 |
| C₁₆H₂₀N₄O₂.HCl | Calc. % | C 60.23 | H 7.18 | N 14.79 |

### N-(endo-8-methyl-8-azabicyclo[3.2.1]oct.-3-yl)-2,3-dihydro-3-propyl-2-oxo-1H-benzimidazole-1-carboxamide, hydrochoric acid salt

### (Compound 7)

M.p. 116-119° C

| | | | | |
|---|---|---|---|---|
| Analysis | Found % | C 59.54 | H 7.23 | N 14.44 |
| C₁₆H₂₀N₄O₂.HCl | Calc. % | C 60.23 | H 7.18 | N 14.79 |

### N-(endo-8-methyl-8-azabicyclo[3.2.1]oct.-3-yl)-2,3-dihydro-3-[(2-methyl)propyl]-2-oxo-1H-benzimidazole-1-carboxamide, hydrochloric acid salt

### (Compound 8)

M.p. 169-170° C

| | | | | |
|---|---|---|---|---|
| Analysis | Found % | C 60.93 | H 7.37 | N 14.36 |
| C₁₆H₂₀N₄O₂.HCl | Calc. % C | 61.14 | H 7.44 | N 14.26 |

### N-(endo-8-methyl-8-azabicyclo[3.2.1]oct.-3-yl)-2,3-dihydro-3-hexyl-2-oxo-1H-benzimidazole-1-carboxamide, hydrochloric acid salt

### (Compound 9)

M.p. 214-215° C

| | | | | |
|---|---|---|---|---|
| Analysis | Found % | C 62.53 | H 7.87 | N 13.22 |
| C₁₆H₂₀N₄O₂.HCl | Calc. % | C 62.77 | H 7.90 | N 13.31 |

### N-(endo-8-methyl-8-azabicyclo[3.2.1]oct.-3-yl)-2,3-dihydro-3-(2-propin-1-yl)-2-oxo-1H-benzimidazole-1-carboxamide, hydrochloric acid salt

### (Compound 10)

M.p. 256-257° C

| | | | | |
|---|---|---|---|---|
| Analysis | Found % | C 60.86 | H 6.36 | N 14.97 |
| C₁₆H₂₀N₄O₂.HCl | Calc. % | C 60.88 | H 6.18 | N 14.95 |

### N-(endo-8-methyl-8-azabicyclo[3.2.1]oct.-3-yl)-2,3-dihydro-3-(3-methyl-2-buten-1-yl)-2-oxo-1H-benzimidazole-1- carboxamide, hydrochloric acid salt

### (Compound 11)

M.p. 196-198° C

| | | | | |
|---|---|---|---|---|
| Analysis | Found % | C 61.53 | H 7.32 | N 13.81 |
| C₁₆H₂₀N₄O₂.HCl | Calc. % | C 62.29 | H 7.22 | N 13.84 |

### N-(endo-8-methyl-8-azabicyclo[3.2.1]oct.-3-yl)-2,3-dihydro-3-methyl-2-oxo-1H-benzimidazole-1-carboxamide, hydrochloric acid salt

### (Compound 12)

M.p. 270° C

| | | | | |
|---|---|---|---|---|
| Analysis | Found % | C 58.14 | H 6.49 | N 16.01 |
| C₁₆H₂₀N₄O₂.HCl | Calc. % | C 58.19 | H 6.61 | N 15.97 |

The following examples describe the incorporation of the active ingredient of formula I into the conventional pharmaceutical compositions for use according to the present invention.

### EXAMPLE 8

| Tablets (direct compression) | |
|---|---|
| - Active ingredient of formula I | 0.50 mg |
| - lactose spray dried | 67.25 mg |
| - microcrystalline cellulose | 21.80 mg |
| - magnesium stearate BP | 0.45 mg |
| Compression weight | 9̅0̅.̅0̅0̅ m̅g̅ |

Method of preparation: the active ingredient was passed through a 60 mesh sieve, blended with the lactose, microcrystalline cellulose and magnesium stearate. The resulting mixture was pressed into tables weight 90 mg each. Each tablet contains 0.50 g of active ingredient.

### EXAMPLE 9

| Capsules | |
|---|---|
| - active ingredient of formula I | 0.50 mg |
| - lactose | 98.50 mg |
| - magnesium stearate BP | 1.00 mg |
| Fill Weight | 1̅0̅0̅.̅0̅0̅ m̅g̅ |

Method of preparation: the active ingredient was sieved and blended with the excipients. The mixture was filled into hard gelatin caspules using suitable machinary.

### EXAMPLE 10

| Syrup | |
|---|---|
| - active ingredient of formula I | 0.50 mg |
| - hydroxypropylmethylcellulose USP | 22.50 mg |
| - Buffer | |
| - Flavour | |
| - Colour | as required |
| - Preservatives | |
| - Sweetener | |
| - Purified water BP | to 5.0 ml |

Method of preparation: the hydroxypropylmethylcellulose was dispersed in hot water, cooled and then mixed with an aqueous solution containing the active ingredient and the other components of the formulation. The resultant solution was adjusted to volume and mixed. The syrup was clarified by filtration.

### EXAMPLE 11

| Ampoules | | |
|---|---|---|
| - active ingredient of formula I | 0.05 mg | 0.5 mg |
| - sodium chloride BP | as required | as required |
| - water for injection BP to | 1.0 ml | 1.0 ml |

Sodium chloride may be added to adjust the tonicity of the solution and the pH may be adjusted, using acid or alkali, to that of optimum stability and/or facilitate solution of the active ingredient. Alternatively suitable buffer salts may be used. Method of preparation: the solution was prepared, clarified and filled into appropriate size ampoules sealed by fusion of the glass. The injection was sterilised by heating in an autoclave using one of the acceptable cycles. Alternatively the solution may be sterilised by filtration and filled into sterile ampoules under aseptic conditions. The solution may be packed under an inert atmosphere of nitrogen or other suitable gas.

### EXAMPLE 12

| Suppositories | |
|---|---|
| - active ingredient of formula I | 0.50 mg |
| - witepsol H35 to | 1.0 g |

Method of preparation: a suspension of the active ingredient was prepared in the molten witepsol and filled, using suitable machinary, into suppository moulds.

## Claims

1. Use of a compound of general formula (I) 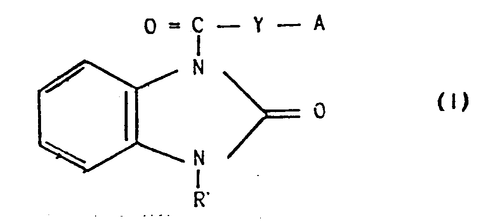 wherein R represents hydrogen atom, C₁₋₈ alkyl, C₂₋₆ alkenyl or C₂₋₆ alkynyl, Y is NH, A is 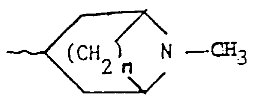 wherein n is 2; and physiologically acceptable acid addition salts and solvates thereof for the manufacture of a medicament for the treatment of organic mental diseases.

2. Use of a compound of general formula I selected from: N-(endo-8-methyl-8-azabicyclo[3.2.1]oct-3-yl)-2,3-dihydro-2-oxo-1H-benzimidazole-1-carboxamide.
N-(endo-8-methyl-8-azabicyclo[3.2.1]oct-3-yl)-2,3-dihydro-3-ethyl-2-oxo-1H-benzimidazole-1-carboxamide.
N-(endo-8-methyl-8-azabicyclo[3.2.1]oct-3-yl)-2,3-dihydro-3-(1-methyl)ethyl-2-oxo-1H-benzimidazole-1-carboxamide
and physiologically acceptable acid salts and solvates thereof for the manufacture of a medicament for the treatment of organic mental diseases.

3. Use of a compound of general formula (I) according to claims 1 or 2 for the manufacture of a medicament for the treatment of primary degeneration dementia and amnestic syndromes.

## Patentansprüche

1. Verwendung einer Verbindung der allgemeinen Formel (I) 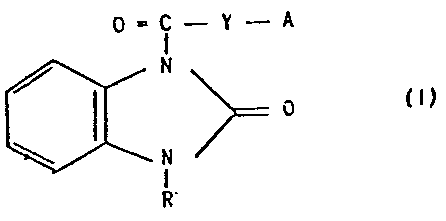 worin R für ein Wasserstoffatom, C₁₋₈-Alkyl, C₂₋₆-Alkenyl oder C₂₋₆-Alkinyl steht, Y für NH steht, A für 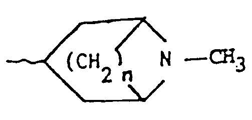 steht, worin n für 2 steht, und der physiologisch akzeptablen Säureadditionssalze und Solvate davon zur Herstellung eines Medikamentes zur Behandlung organischer Geisteskrankheiten.

2. Verwendung einer Verbindung der allgemeinen Formel I, ausgewählt unter:
N-(Endo-8-methyl-8-azabicyclo[3.2.1]oct-3-yl)-2,3-dihydro-2-oxo-1H-benzimidazol-1-carboxamid;
N-(Endo-8-methyl-8-azabicyclo[3.2.1]oct-3-yl)-2,3-dihydro-3-ethyl-2-oxo-1H-benzimidazol-1-carboxamid.
N-(Endo-8-methyl-8-azabicyclo[3.2.1]oct-3-yl)-2,3-dihydro-3-(1-methyl)ethyl-2-oxo-1H-benzimidazol-1-carboxamid
und der physiologisch akzeptablen Säuresalze und Solvate davon zur Herstellung eines Medikamentes zur Behandlung organischer Geisteskrankheiten.

3. Verwendung einer Verbindung der allgemeinen Formel (I) gemäß Anspruch 1 oder 2 zur Herstellung eines Medikamentes zur Behandlung primärer degenerativer Demenz und amnestischer Syndrome.

## Revendications

1. Utilisation d'un composé de formule générale (I) 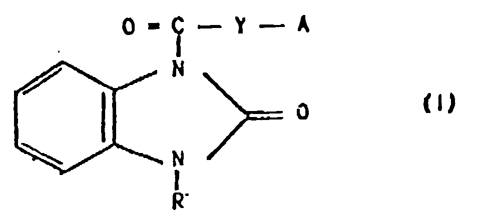 dans laquelle R représente un atome d'hydrogène, un alkyle en C₁₋₈, un alcényle en C₂₋₆ ou un alcynyle en C₂₋₆, Y est NH, A est 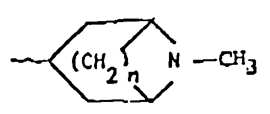 où n est 2 ; et de ses sels d'addition d'acides et solvates physiologiquement acceptables, pour la préparation d'un médicament pour le traitement des maladies mentales organiques.

2. Utilisation d'un composé de formule générale (I) choisi parmi :
le N-(endo-8-méthyl-8-azabicyclo[3.2.1]oct-3-yl)-2,3-dihydro-2-oxo-1H-benzimidazole-1-carboxamide
le N-(endo-8-méthyl-8-azabicyclo[3.2.1]oct-3-yl)-2,3-dihydro-3-éthyl-2-oxo-1H-benzimidazole-1-carboxamide
le N-(endo-B-méthyl-8-azabicyclo[3.2.1]oct-3-yl)-2,3-dihydro-3-(1-méthyl)éthyl-2-oxo-1H-benzimidazole-1-carboxamide
et leurs sels et solvates physiologiquement acceptables, pour la préparation d'un médicament pour le traitement des maladies mentales organiques.

3. Utilisation d'un composé de formule générale (I) selon les revendications 1 ou 2 pour la préparation d'un médicament pour le traitement de la démence dégénérative primaire et des syndromes d'amnésie.
